# EUROPEAN PATENT SPECIFICATION

(11) **EP 3 981 756 B1**
(45) Date of publication and mention of the grant of the patent: **02.07.2025**
(21) Application number: 20819214.6
(22) Date of filing: 19.05.2020
(51) Int. Cl.: C09K 5/04, C09K 3/30, C23G 5/028, C11D 7/30, C11D 7/50, C07C 21/073, C07C 21/18

(54) **AZEOTROPIC COMPOSITION, AZEOTROPE-LIKE COMPOSITION, COMPOSITION, CLEANING AGENT, SOLVENT, AND HEAT TRANSFER MEDIUM**
AZEOTROPE ZUSAMMENSETZUNG, AZEOTROPARTIGE ZUSAMMENSETZUNG, ZUSAMMENSETZUNG, REINIGUNGSMITTEL, LÖSUNGSMITTEL UND WÄRMEÜBERTRAGUNGSMEDIUM
COMPOSITION AZÉOTROPIQUE, COMPOSITION DE TYPE AZÉOTROPE, COMPOSITION, AGENT DE NETTOYAGE, SOLVANT ET MILIEU CALOPORTEUR

(30) Priority: 07.06.2019 JP 2019107140
(43) Date of publication of application: 13.04.2022
(73) Proprietor: AGC INC., Chiyoda-ku, Tokyo 1008405 (JP)
(72) Inventor: MITSUOKA Hiroaki, Tokyo 100-8405 (JP)
(74) Representative: Müller-Boré & Partner Patentanwälte PartG mbB
(86) International application number: PCT/JP2020/019820
(87) International publication number: WO 2020/246231

(56) References cited:
- WO-A1-2018/097300
- WO-A1-2018/101324
- WO-A1-2019/039521
- WO-A1-2020/142650
- US-A1- 2013 004 435
- US-A1- 2015 231 527

## Description

### TECHNICAL FIELD

The present invention relates to an azeotropic composition, an azeotrope-like composition and a composition, which have little influence over the global environment, which are non-flammable, and which are less likely to undergo composition change even when evaporated and condensed repeatedly, and a detergent, a solvent and a heat transfer fluid, which contain the azeotropic composition, the azeotrope-like composition or the composition.

### BACKGROUND ART

Heretofore, chlorofluorocarbons (hereinafter referred to as "CFC"), hydrochlorofluorocarbons (hereinafter referred to as "HCFC") and hydrofluorocarbons (hereinafter referred to as "HFC"), which are inflammable and are excellent in stability, drying property, etc., are used for various applications including detergents, solvents, heat transfer fluids, blowing agents, fire extinguishing agents, etc. However, influences of CFCs, HCFCs and HFCs over the ozone layer and global warming have been of concern.

As alternatives to CFCs, HCFCs and HFCs, fluoroolefins have been proposed. Fluoroolefins, which have a carbon-carbon double bond in their molecules, have a short lifetime in the atmosphere and little influence over the global environment.

However, if a fluoroolefin is used for the above application by itself, required performance may sometimes not sufficiently be developed. To overcome such a problem, use of other component in combination with a fluoroolefin depending upon the aimed purpose has been studied.

For example, Patent Document 1 proposes a composition containing monochlorotrifluoropropene (HCFO-1233) and an additional component.

### PRIOR ART DOCUMENTS

### PATENT DOCUMENTS

Patent Document 1: WO2011/031697

US 2015/231527 describes a composition containing monochlorotrifluoropropene compounds.

US 2013/004435 relates generally to compositions comprising 1-chloro-3,3,3-trifluoropropene.

### DISCLOSURE OF INVENTION

### TECHNICAL PROBLEM

However, in a case where two or more components are used in combination, due to a difference in the boiling point between the respective components, the composition may change during use and no sufficient performance may sometimes be developed. Further, if a component having a flash point is used in combination as an additional component, it is necessary to take countermeasures for safety.

The present invention is to provide an azeotropic composition, an azeotrope-like composition and a composition, which have little influence over the global environment, which are non-flammable, and which are less likely to undergo composition change even when evaporated and condensed repeatedly.

The present invention further provides a detergent, a solvent and a heat transfer fluid, which contain the azeotropic composition, the azeotrope-like composition or the composition.

### SOLUTION TO PROBLEM

The present inventor has conducted extensive studies and as a result, accomplished the present invention. That is, the present invention provides the subject matter of claims 1 to 9.

### ADVANTAGEOUS EFFECTS OF INVENTION

The azeotropic composition, the azeotrope-like composition and the composition of the present invention have little influence over the global environment, are non-flammable, and are less likely to undergo composition change even when evaporated and condensed repeatedly.

The detergent, the solvent and the heat transfer fluid of the present invention have little influence over the global environment, are non-flammable, and are less likely to undergo composition change even when evaporated and condensed repeatedly.

### BRIEF DESCRIPTION OF DRAWINGS

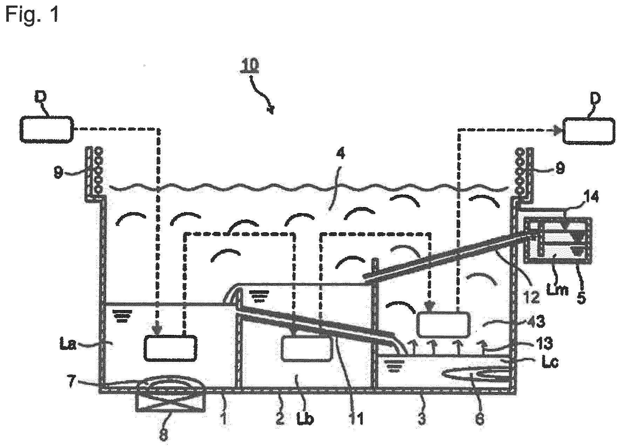

Fig. 1 is a view schematically illustrating a cleaning apparatus to conduct the cleaning method of the present invention.

### DESCRIPTION OF EMBODIMENTS

In this specification, with respect to halogenated hydrocarbons, compound names are accompanied by abbreviated names in brackets, and in this specification, as the case requires, the abbreviated names are used instead of the compound names. Further, as the abbreviated names, only numerals and alphabet small letters after the hyphen (-) may sometimes be used (for example, "1233yd" in the case of "HCFO-1233yd"). Further, (E) accompanying names of compounds having geometric isomers and their abbreviated names represent E-isomer, and (Z) represent Z-isomers. Compound names and abbreviated names not accompanied by (E) or (Z), generally represent E-isomer, Z-isomer and a mixture of E-isomer and Z-isomer.

In this specification, a compound having one or more hydrogen atoms in a saturated hydrocarbon compound replaced by a fluorine atom, will be referred to as a hydrofluorocarbon (HFC), a compound having one or more hydrogen atoms in a saturated hydrocarbon compound replaced by a fluorine atom and a chlorine atom, as a hydrochlorofluorocarbon (HCFC), a compound having all hydrogen atoms in a saturated hydrocarbon compound replaced by a fluorine atom and a chlorine atom, as a chlorofluorocarbon (CFC), a compound composed of carbon atoms, fluorine atoms and hydrogen atoms, having a carbon-carbon double bond, as a hydrofluoroolefin (HFO), and a compound composed of carbon atoms, chlorine atoms, fluorine atoms and hydrogen atoms, having a carbon-carbon double bond, as a hydrochlorofluoroolefin (HCFO).

In this specification, a range of numerical values represented by " to " is used to include the numerical values before and after it as the lower limit value and the upper limit value.

### [Azeotropic composition]

The azeotropic composition of the present invention consists essentially of 34.5 mass% of (Z)-1-chloro-2,3,3-trifluoropropene (HCFO-1233yd(Z)) and 65.5 mass% of trans-1,2-dichloroethylene (hereinafter referred to as "tDCE"). That is, in the azeotropic composition of the present invention, the 1233yd(Z) content to the total amount of the azeotropic composition of the present invention is 34.5 mass%, and the tDCE content to the total amount of the azeotropic composition of the present invention is 65.5 mass%.

The boiling point of the azeotropic composition of the present invention under a pressure of 1.0×10⁵ Pa is 46°C.

The azeotropic composition of the present invention has little influence over the global environment, is non-flammable, and is less likely to undergo composition change even when evaporated and condensed repeatedly, and accordingly has such advantages that when used for various applications such as detergents, solvents and heat transfer fluids, it exhibits very stable performance.

The azeotropic composition has a relative volatility represented by the following formula of 1.00.

### (Formula to calculate relative volatility)

Relative volatility=(mass% of 1233yd(Z) in liquid phase portion/mass% of tDCE in liquid phase portion)/(mass% of 1233yd(Z) in gaseous phase portion/mass% of tDCE in gaseous phase portion)

### [Azeotrope-like composition]

The azeotrope-like composition consists essentially of 1233yd(Z) and tDCE of the present invention is a composition having a proportion of 1233yd(Z) of from 28 to 41 mass% and a proportion of tDCE of from 59 to 72 mass%. That is, in the azeotrope-like composition of the present invention, the 1233yd(Z) content to the total amount of the azeotrope-like composition of the present invention is from 28 to 41 mass%, and the tDCE content to the total amount of the azeotrope-like composition of the present invention is from 59 to 72 mass% to the total amount of the azeotrope-like composition of the present invention.

In this specification, the azeotrope-like composition means a composition having a relative volatility obtained by the above formula within a range of 1.00±0.10.

Further, the boiling point of the azeotrope-like composition of the present invention under a pressure of 1.0×10⁵ Pa is from 44 to 48°C.

The azeotrope-like composition of the present invention has little influence over the global environment, is non-flammable, and is less likely to undergo composition change even when evaporated and condensed repeatedly, and can be dealt with in substantially the same manner as the azeotropic composition of the present invention. Accordingly, it has such advantages that it exhibits a stable performance comparable to the azeotropic composition when used for various applications such as detergents, solvents and heat transfer fluids.

### [Composition]

The composition of the present invention is a composition containing 1233yd(Z) and tDCE, wherein the total proportion of 1233yd(Z) and tDCE in the composition to the total amount of the composition is at least 90 mass%, and the mass ratio of 1233yd(Z) to tDCE (1233yd(Z)/tDCE) is from 28/72 to 41/59.

That is, the composition of the present invention contains 1233yd(Z) and tDCE in a total amount of at least 90 mass% to the total amount of the composition.

The composition of the present invention may contain, within a range not to impair the effects of the present invention, components other than 1233yd(Z) and tDCE (hereinafter sometimes referred to as "other component") in an amount of at most 10 mass% to the total amount of the composition.

As other component, (E)-1-chloro-2,3,3-trifluoropropene(HCFO-1233yd(E)), 1-chloro-2,2,3,3-tetrafluoropropane (HCFC-244ca), 1,1,2-trifluoro-3-chloropropene (HCFO-1233yc), 1-chloro-3,3-difluoropropyne, trichloroethylene, and cis-1,2-dichloroethylene may, for example, be mentioned.

The content of other component to the total amount of the composition is preferably at most 7 mass%, more preferably at most 5 mass%.

The boiling point of the composition of the present invention under a pressure of 1.0×10⁵ Pa is from 44 to 48°C.

The composition of the present invention, which contains 1233yd(Z) and tDCE in a total amount of at least 90 mass%, has little influence over the global environment, is non-flammable, and is less likely to undergo composition change even when evaporated and condensed repeatedly, and accordingly, can be dealt with in substantially the same manner as the azeotropic composition or the azeotrope-like composition of the present invention. Accordingly, it has such advantages that it exhibits stable performance comparable to the azeotropic composition or the azeotrope-like composition when used for various applications such as detergents, solvents and heat transfer fluids.

1233yd(Z), which is an olefin having a carbon-carbon double bond, has a short lifetime in the atmosphere, and low ozone depletion coefficient and low global warming potential.

1233yd(Z) has a boiling point of 54°C and can be used even for members which are susceptible to heat. Further, 1233yd(Z) has excellent performance as a detergent or a solvent, such as having no flash point, being excellent in drying property, and having low surface tension and viscosity and thereby excellent permeability.

Further, 1233yd(Z), which has a low viscosity, has small resistance when it passes through a piping, is excellent in transport properties, and has excellent performance as a heat transfer fluid.

1233yd(Z) may be produced, for example, by the method described in WO2017/018412. By the above method, usually, an isomer mixture of 1233yd(Z) and 1233yd(E) is obtained. 1233yd(Z) is obtained by purifying the isomer mixture by a known method such as distillation.

The azeotropic composition, the azeotrope-like composition and the composition of the present invention, which contain 1233yd(Z) respectively in the above amounts, have little influence over the global environment, are non-flammable, and exhibit performance suitable for various applications such as detergents, solvents and heat transfer fluids.

tDCE, which is an olefin having a carbon-carbon double bond, has a short lifetime in the atmosphere and little influence over the global environment. tDCE, which has a chlorine atom in its molecule, provides very high solubility to organic matters.

tDCE has a boiling point of about 49°C and can be used even for members which are susceptible to heat.

Further, tDCE has excellent performance as a detergent or a solvent, such as being excellent in drying property, and having low surface tension and viscosity and thereby excellent permeability.

Further, tDCE, which has a low viscosity, has small resistance when it passes through a piping, is excellent in transport properties, and has excellent performance as a heat transfer fluid.

On the other hand, tDCE has a problem such that it has a flash point.

The azeotropic composition, the azeotrope-like composition and the composition of the present invention, which contain tDCE, are excellent in various performance such as cleaning property, solubility and transfer property. Further, they have a lower boiling point than in a case of using 1233yd(Z) by itself.

The above-described azeotropic composition, the azeotrope-like composition and the composition of the present invention have little influence over the global environment, are non-flammable, and are less likely to undergo composition change even when evaporated and condensed repeatedly, and exhibit excellent performance suitable for various applications such as detergents, solvents and heat transfer fluids.

The azeotropic composition, the azeotrope-like composition and the composition of the present invention are used preferably as detergents, solvents, aerosol compositions, heat transfer fluids, fire extinguishing agents, blowing agents, and the like.

As a material to which the azeotropic composition, the azeotrope-like composition and the composition of the present invention are applicable, metals, plastics, elastomers, glass, ceramics, fibers and composite materials thereof may, for example, be mentioned. Among them, metals such as iron, copper, nickel, gold, silver and platinum, metal sintered products, glass, and engineering plastics such as fluororesin, polyimide, polyphenylene sulfide, liquid crystal polymers and PEEK are suitable.

### [Detergent]

The azeotropic composition, the azeotrope-like composition and the composition of the present invention, which are excellent in cleaning property, are useful as a detergent for degreasing cleaning, flux cleaning, precision cleaning, dewater cleaning, rinse cleaning, dry cleaning, and the like.

As an article to which the detergent of the present invention is applicable, optical articles, medical equipment, electrical equipment, precision machinery, fiber products, and members thereof. As the electrical equipment, precision machinery, optical articles and members thereof, ICs, condensers, printed boards, micromotors, relays, bearings, optical lenses, glass substrates and the like may be mentioned. As the medical equipment, catheters, needles and the like may be mentioned.

As a method for cleaning an article, a method of brining the detergent of the present invention into contact with an article to remove stains attached to the article may be mentioned.

The stains attached to an article may, for example, be grease, process oils, silicone oils, fluxes, waxes, inks, mineral oils, release agents containing silicone oil, fats and oils such as pitch and asphalt, and dust. The process oil may, for example, be cutting oil, quenching oil, rolling oil, lubricating oil, machine oil, press oil, punching oil, drawing oil, assembling oil or wire drawing oil.

The detergent of the present invention is excellent in cleaning property and is thereby suitable particularly for cleaning off process oil, pitch and asphalt.

As a method for cleaning an article, manual cleaning, immersion cleaning, spray cleaning, immersion oscillating cleaning, immersion ultrasonic cleaning, steam cleaning, and a combination thereof may, for example, be mentioned.

The cleaning conditions in the cleaning method, such as the contact time with the article and the temperature may properly be selected depending upon the cleaning method. Further, as a cleaning apparatus, a known one may be selected. For example, the method and the cleaning apparatus described in WO2008/149907 may be employed.

The detergent of the present invention contains the azeotropic composition, the azeotrope-like composition or the composition. The content of the azeotropic composition, the azeotrope-like composition or the composition of the present invention in the detergent of the present invention, to the tola amount of the detergent, preferably at least 80 mass%, more preferably at least 90 mass%, further preferably at least 95 mass%. The upper limit may be 100 mass%.

The detergent of the present invention may contain a stabilizer of the like as the case requires.

The stabilizer may, for example, be nitromethane, nitroethane, nitropropane, nitrobenzene, diethylamine, triethylamine, isopropylamine, diisopropylamine, butylamine, isobutylamine, tert-butylamine, α-picoline, N-methylbenzylamine, diallylamine, N-methylmorpholine, phenol, o-cresol, m-cresol, p-cresol, thymol, p-tertbutylphenol, tert-butylcatechol, catechol, isoeugenol, o-methoxyphenol, p-methoxyphenol, 4,4'-dihydroxyphenyl-2,2-propane, isoamyl salicylate, benzyl salicylate, methyl salicylate, 2,6-di-tert-butyl-p-cresol, 2-(2'-hydroxy-5'-methylphenyl)benzotriazole, 2-(2'-hydroxy-3'-tert-butyl-5'-methylphenyl)-5-chlorobenzotriazole, 1,2,3-benzotriazole, 1-[(N,N-bis-2-ethylhexyl)aminomethyl]benzotriazole, 1,2-propylene oxide, 1,2-butylene oxide, 1,4-dioxane, butyl glycidyl ether, phenyl glycidyl ether, isoamylene, 2,4,4-trimethyl-1-pentene, 2,4,4-trimethyl-2-pentene or 2-methylpentene. Among them, 2,6-di-tert-butyl-p-cresol, p-methoxyphenol, 1,2-butylene oxide, isoamylene, 2,4,4-trimethyl-1-pentene, 2,4,4-trimethyl-2-pentene and 2-methylpentene are preferred.

The stabilizer may be used alone or in combination of two or more.

The content of the stabilizer in the detergent of the present invention, to the total amount of the detergent, is preferably at most 5 mass%, more preferably at most 1 mass%.

### [Solvent]

The azeotropic composition, the azeotrope-like composition and the composition of the present invention provide excellent solubility to organic matters and are thereby useful as a solvent in which a surface treatment agent such as a lubricating oil, an anticorrosive agent, a moisture-proof coating agent or an anti-fouling coating agent is dissolved to be applied to the surface of an article.

As the article, the same articles as ones to which the detergent is applicable, may be mentioned.

As a method of coating the surface of an article with the surface treatment agent, the surface treatment agent is dissolved in the solvent of the present invention, and the solvent is evaporated to form a coating film on the surface of an article.

When the surface treatment agent is dissolved in the solvent of the present invention, it is preferred to dissolve the surface treatment agent so that its concentration will be from 0.01 to 50 mass%.

As a coating method, coating by a brush, coating by spraying and coating by dipping may, for example, be mentioned. In a case where the article is tubular, a surface treatment agent solution obtained by dissolving the surface treatment agent in the solvent may be sucked up to be applied to the inner wall, in some cases. Further, the surface treatment agent, the solvent of the present invention and a liquefied gas or compressed gas are sealed to form an aerosol, which is applied by spraying. Specific examples of the liquified gas and the compressed gas are as described above.

As a method of evaporating the solvent, air drying and drying by heating may, for example, be mentioned. The drying temperature is preferably from 20 to 100°C.

The solvent of the present invention contains the azeotropic composition, the azeotrope-like composition or the composition. The content of the azeotropic composition, the azeotrope-like composition or the composition of the present invention in the solvent of the present invention, to the total amount of the solvent, is preferably at least 80 mass%, more preferably at least 90 mass%, further preferably at least 95 mass%. The upper limit may be 100 mass%.

The solvent of the present invention may contain a stabilizer or the like as the case requires. As specific examples of the stabilizer, the above-described stabilizers may be mentioned.

The content of the stabilizer in the solvent of the present invention, to the total amount of the solvent, is preferably at most 5 mass%, more preferably at most 1 mass%.

### [Aerosol composition]

The azeotropic composition, the azeotrope-like composition and the composition of the present invention may be used as an aerosol as combined with an aerosol propellant. The pressure of the aerosol propellant used in the present invention is preferably at least atmospheric pressure (1.013×10⁵ Pa) at 0°C.

In the present composition, the aerosol propellant may, for example, be dimethyl ether (DME), propane, butane, isobutane, 1,1-difluoroethane (HFC-152a), 1,1,1,2-tetrafluoroethane (HFC-134a), 2,3,3,3-tetrafluoropropene (HFO-1234yf), 1,3,3,3-tetrafluoropropene (HFO-1234ze), nitrogen, carbon dioxide, or nitrous oxide, and they may be used alone or in combination of two or more. For example, e.g. air may preferably be used.

As the aerosol propellant, either of liquified gas or compressed gas may be used, and liquified gas and compressed gas may be used in combination.

The aerosol propellant contained in the aerosol composition exists preferably under a pressure of from 0.3 to 1MPa at 35°C when contained in a sprayer.

### [Heat transfer fluid]

The azeotropic composition, the azeotrope-like composition and the composition of the present invention may be used as a heat transfer fluid for heat cycle system.

The heat cycle system may, for example, be a Rankine cycle system, a heat pump cycle system, a refrigerating cycle system, a heat transfer system or a secondary circulative cooling system. More specifically, a refrigerator, an air-conditioning apparatus, a power generation system, a heat transfer apparatus and a secondary cooling machine may, for example, be mentioned.

Now, a refrigerating cycle system as an example of the heat cycle system will be described.

The refrigerating cycle system is a system wherein in an evaporator, a heat transfer fluid removes heat energy from a load fluid to cool the load fluid thereby to accomplish cooling to a lower temperature. The refrigerating cycle system is a system comprising a compressor to compress a vapor of the heat transfer fluid to form a high temperature/high pressure vapor of the heat transfer fluid, a condenser to cool the compressed vapor of the heat transfer fluid and to form a low temperature/high pressure liquid of the heat transfer fluid, an expansion valve to let the liquid of the heat transfer fluid discharged from the condenser expand to form a low temperature/low pressure liquid of the heat transfer fluid, an evaporator to heat the heat transfer fluid discharged from the expansion valve to form a high temperature/low pressure vapor of the heat transfer fluid, a pump to supply a load fluid to the evaporator, and a pump to supply a load fluid to the condenser.

The azeotropic composition, the azeotrope-like composition and the composition of the present invention are useful particularly preferably as a secondary refrigerant for a secondary circulative cooling system.

The secondary circulative cooling system is a system having a primary cooling means to cool a primary refrigerant comprising ammonia or hydrocarbon refrigerant, a secondary circulative cooling means to circulate a secondary refrigerant for secondary circulative cooling system to cool an object to be cooled, and a heat exchanger to conduct heat exchange between the primary refrigerant and the secondary refrigerant to cool the secondary refrigerant. By such a secondary circulative cooling system, the object to be cooled can be cooled.

The heat transfer fluid of the present invention contains the azeotropic composition, the azeotrope-like composition or the composition. The content of the azeotropic composition, the azeotrope-like composition or the composition of the present invention in the heat transfer fluid of the present invention, to the total amount of the heat transfer fluid, is preferably at least 80 mass%, more preferably at least 90 mass%, further preferably at least 95 mass%. The upper limit may be 100 mass%.

The heat transfer fluid of the present invention may contain a stabilizer and the like as the case require. As specific examples of the stabilizer, the above described stabilizers may be mentioned.

The content of the stabilizer in the solvent of the present invention to the total amount of the solvent is preferably at most 5 mass%, more preferably at most 1 mass%.

The heat transfer fluid of the present invention is used usually as a heat transfer fluid composition containing the heat transfer fluid and a refrigerant oil. The refrigerant oil may, for example, be a polyalkylene glycol, a polyol ester or a polyvinyl ether.

The content of the refrigerant oil in the heat transfer fluid composition per 100 parts by mass of the heat transfer fluid is preferably from 10 to 100 parts by mass, more preferably from 20 to 50 parts by mass.

### EXAMPLES

Now, the present invention will be described in further detail with reference to Examples. However, it should be understood that the present invention is by no means restricted thereto. Ex. 1 to 8 are Examples of the present invention, and Ex. 9 to 11 are Comparative Examples.

### <Examples for production of 1233yd>

1233yd was produced by the method described in Example 1 of WO2017/018412. The isomer mixture of 1233yd(Z) and 1233yd(E) obtained by the above method was purified by distillation to obtain 1233yd(Z) and 1233yd(E).

### <Measurement (1) of liquid-vapor equilibrium>

300 g of each of mixtures 1 to 12 obtained by mixing 1233yd(Z) and tDCE in a mass ratio as identified in Table 1 was distilled in an Othmar liquid-vapor equilibrium distilling apparatus (manufactured by SHIBATA SCIENTIFIC TECHNOLOGY LTD.). The pressure in the apparatus was atmospheric pressure (1.0×10⁵ Pa). After the temperatures in the gaseous phase and the liquid phase were confirmed to be unchanged for 1 hour and stabilized, samples were collected from the gaseous phase and the liquid phase in the apparatus, and the mass ratios of 1233yd(Z) to tDCE were measured by gas chromatography. From the mass ratios of 1233yd(Z) to tDCE in the gaseous phase and the liquid phase, the relative volatility was calculated from the above formula to calculate the relative volatility. The results are shown in Table 1.

**[Table 1]**

| Mixture | Mixture (mass%) | | Liquid phase (mass%) | | Gaseous phase (mass%) | | Relative volatility |
|---|---|---|---|---|---|---|---|
| | 1233yd(Z) | tDCE | 1233yd(Z) | tDCE | 1233yd(Z) | tDCE | |
| 1 | 10 | 90 | 9.6 | 90.4 | 13.9 | 86.1 | 0.66 |
| 2 | 20 | 80 | 19.6 | 80.4 | 23.7 | 76.3 | 0.78 |
| 3 | 28 | 72 | 27.8 | 72.2 | 29.9 | 70.1 | 0.90 |
| 4 | 30 | 70 | 29.8 | 70.2 | 31.3 | 68.7 | 0.93 |
| 5 | 34.5 | 65.5 | 34.5 | 65.5 | 34.5 | 65.5 | 1.00 |
| 6 | 40 | 60 | 40.2 | 59.8 | 38.1 | 61.9 | 1.09 |
| 7 | 41 | 59 | 41.0 | 59.0 | 38.7 | 61.3 | 1.10 |
| 8 | 50 | 50 | 50.6 | 49.4 | 44.8 | 55.2 | 1.26 |
| 9 | 60 | 40 | 60.9 | 39.1 | 51.8 | 48.2 | 1.45 |
| 10 | 70 | 30 | 71.1 | 28.9 | 59.7 | 40.3 | 1.66 |
| 11 | 80 | 20 | 81.2 | 18.8 | 69.3 | 30.7 | 1.91 |
| 12 | 90 | 10 | 90.8 | 9.2 | 82.1 | 17.9 | 2.15 |

From Table 1, in mixtures in a mass ratio of 1233yd(Z) to tDCE of from 28/72 to 41/59, the relative volatility was 1.00±0.10.

### <Measurement (2) of liquid-vapor equilibrium>

300 g of mixture 13 having a mass ratio of 1233yd(Z), 1233yd(E) and tDCE (1233yd(Z)/1233yd(E)/tDCE) of 31.2/1.3/67.5, was distilled in an Othmar liquid-vapor equilibrium distilling apparatus (manufactured by SHIBATA SCIENTIFIC TECHNOLOGY LTD.). The pressure in the apparatus was atmospheric pressure (1.0×10⁵ Pa). Samples were collected from the gaseous phase and the liquid phase in the apparatus, and the composition ratios of 1233yd(Z), 1233yd(E) and tDCE were measured by gas chromatography, whereupon the composition ratio in the liquid phase was 31.2/1.2/67.6, and the composition ratio in the gaseous phase was 31.1/1.5/67.4, and the compositions in the liquid phase and in the gaseous phase were substantially the same.

### <Evaluation of Performance>

### (1) Metal process oil cleaning test

A SUS-304 test piece (25 mm × 30 mm × 2 mm) was dipped in a metal process oil (product name: EM-7451 (dynamic viscosity: 342 mm²/s, 40°C), manufactured by Nihon Kohsakuyu Co., Ltd.) to prepare a metal process oil-attached test piece. The metal process oil-attached test specimen piece was dipped in 50 mL of each of the compositions in Ex. 1 to 11 as identified in Tables 2 and 3 and pulled up, and the state of the remaining metal process oil on the test piece was visually observed and taken as the standard for evaluation of cleaning property. The test was conducted at a temperature of 23°C, and "A" represents a case where the metal process oil was removed, and "B" represents a case where the metal process oil remained. The results are shown in "Test (1)" in Tables 2 and 3.

### (2) Pitch cleaning test

On a 10 mm × 20 mm × 5 mm glass substrate, spray pitches (trade name: Spray Pitches, manufactured by KOKONOE ELECTRIC CO.,LTD.) were sprayed and dried overnight to prepare a pitch-attached glass substrate test piece. 100 g of each of compositions in Ex. 1 to 11 as identified in Tables 2 and 3 was put in a 100 ml glass beaker, the above test piece was dipped for 1 minute, and the degree of removal of the pitches from the test piece was visually evaluated. "A" represents a case where the pitches were removed from the glass substrate test piece, and "B" represents a case where the pitch component remained on the glass substrate test piece. The results are shown in "Test (2)" in Tables 2 and 3.

### (3) Evaluation of cleaning performance by aerosol composition

A SUS304 test piece (25 mm × 30 mm × 2 mm in thickness) was dipped in a metal process oil (Daphne Magplus LA-5 manufactured by Idemitsu Kosan Co., Ltd.) for one minute, pulled up and left at rest overnight, to prepare a test piece.

Each of the solvent compositions in Ex. 1 to 8 and an aerosol propellant (CO₂ or N₂) in a mass ratio represented by solvent composition/aerosol propellant of 95/5 were contained in a spray can. The solvent composition was sprayed from the spray can, whereupon the metal process oil could be removed from the test piece. Further, the solvent composition attached to the test piece was quickly dried. The cleaning performance was constant regardless of the type of the aerosol propellant.

### (4) Flammability test

200 mL of each of the compositions in Ex. 1 to 11 as identified in Tables 2 and 3 was subjected to Cleveland open-cup flash point tester (manufactured by YOSHIDA MANUFACTURING CO., LTD., model 828) to confirm whether the composition had a flash point or not between 23°C and the boiling point. The results are shown in "Flammability" in Tables 2 and 3.

The rows for the respective components (rows "1233yd(Z)", "1233yd(E)" and "tDCE") in Tables 2 and 3 represent the contents (mass%) of the respective components to the total amount of the composition.

**[Table 2]**

| Ex. | 1 | 2 | 3 | 4 | 5 | 6 |
|---|---|---|---|---|---|---|
| 1233yd(Z) | 28 | 34.5 | 34.2 | 31 | 28 | 41 |
| 1233yd(E) | - | - | 0.3 | 3 | 7 | - |
| tDCE | 72 | 65.5 | 65.5 | 66 | 65 | 59 |
| Test (1) | A | A | A | A | A | A |
| Test (2) | A | A | A | A | A | A |
| Flammability | Nil | Nil | Nil | Nil | Nil | Nil |

**[Table 3]**

| Ex. | 7 | 8 | 9 | 10 | 11 |
|---|---|---|---|---|---|
| 1233yd(Z) | 40.5 | 31 | 10 | 15 | 100 |
| 1233yd(E) | 0.5 | 10 | - | - | - |
| tDCE | 59.0 | 59 | 90 | 85 | - |
| Test (1) | A | A | A | A | B |
| Test (2) | A | A | A | A | B |
| Flammability | Nil | Nil | Observed | Observed | Nil |

### <Cleaning Test>

Each of the compositions obtained in Ex. 12 and 13 in the following Table 4 was applied to the same cleaning apparats as shown in Fig. 1 to conduct cleaning test. This cleaning test is a test to evaluate the following compositions and is an Example of the cleaning method of the present invention. Ex. 12 is an Example of the present invention, and Ex. 13 is a Comparative Example.

Each of the compositions obtained in the following Ex. 12 and 13 was put in all three tanks of the cleaning apparatus 10 shown in Fig. 1, that is in a cleaning tank 1 (capacity: 5.2 L), a rinsing tank 2 (capacity: 5.0 L) and a vapor generating tank 3 (capacity: 2.8 L). The cleaning apparatus 10 was continuously run for 8 hours without conducting cleaning to stabilize the composition in each tank in the cleaning apparatus 10 into a stationary state. Further, as the article D to be cleaned, the same test piece used in Evaluation of Performance (1) and (2) was prepared.

Using the cleaning apparatus 10 in a stationary state, as shown in Fig. 1, the test piece was put into the washing tank 1, the rinsing tank 2 and the vapor generating tank 3 in this order to be cleaned. The temperature of the composition La in the cleaning tank La was 40°C, and in cleaning in the cleaning tank 1, ultrasonic waves at a frequency of 40 kHz at an output of 200 W were generated for 1 hours. Further, the temperature of the composition Lb in the rinsing tank 2 was 25°C, and the composition Lc in the vapor generating tank 3 was heated to be always in a boiling state. During cleaning, the vapor in a vapor zone 4 was condensed and moisture was removed therefrom to obtain composition Lm, which was returned to the rinsing tank 2, the overflow from the rinsing tank 2 was made to flow into the cleaning tank 1, and the composition La in excess in the cleaning tank 1 was transferred to the vapor generating tank 3.

After completion of cleaning, the composition La in the cleaning tank 1 was collected, the composition of which was analyzed by a gas chromatograph (manufactured by Agilent Technologies Japan, Ltd., GC7890). Further, the state of the test piece after cleaned was evaluated in the same manner as in Cleaning Evaluation (1) and (2).

**[Table 4]**

| Ex. | Ex. 12 | Ex. 13 |
|---|---|---|
| Mixture (wt%) | 34.5/65.5 | 50.0/50.0 |
| 1233yd(Z)/tDCE | | |
| Composition La in cleaning tank 1 (wt%) | 34.5/65.5 | 52.0/48.0 |
| 1233yd(Z)/tDCE | | |
| Evaluation of Performance (1) | A | A |
| Evaluation of Performance (2) | A | B |

### INDUSTRIAL APPLICABILITY

The azeotropic composition, the azeotrope-like composition and the composition of the present invention are useful for a variety of applications such as detergents, solvents and heat transfer fluids.

## Claims

1. An azeotropic composition consisting essentially of 34.5 mass% of (Z)-1-chloro-2,3,3-trifluoropropene and 65.5 mass% of trans-1,2-dichloroethylene.

2. An azeotrope-like composition consisting essentially of from 28 to 41 mass% of (Z)-1-chloro-2,3,3-trifluoropropene and from 59 to 72 mass% of trans-1,2-dichloroethylene.

3. The azeotrope-like composition according to Claim 2, which has a relative volatility of 1.00±0.10.

4. A composition containing (Z)-1-chloro-2,3,3-trifluoropropene and trans-1,2-dichloroethylene, wherein the total proportion of (Z)-1-chloro-2,3,3-trifluoropropene and trans-1,2-dichloroethylene in the composition to the total amount of the composition is at least 90 mass%, and the mass ratio of (Z)-1-chloro-2,3,3-trifluoropropene to trans-1,2-dichloroethylene is from 28/72 to 41/59.

5. The composition according to Claim 4, which further contains (E)-1-chloro-2,3,3-trifluoropropene.

6. A detergent containing the azeotropic composition as defined in Claim 1 or the azeotrope-like composition as defined in Claim 2 or 3 or the composition as defined in Claim 4 or 5.

7. A solvent containing the azeotropic composition as defined in Claim 1 or the azeotrope-like composition as defined in Claim 2 or 3 or the composition as defined in Claim 4 or 5.

8. An aerosol composition containing the azeotropic composition as defined in Claim 1 or the azeotrope-like composition as defined in Claim 2 or 3 or the composition as defined in Claim 4 or 5.

9. A heat transfer fluid containing the azeotropic composition as defined in Claim 1 or the azeotrope-like composition as defined in Claim 2 or 3 or the composition as defined in Claim 4 or 5.

## Patentansprüche

1. Azeotrope Zusammensetzung, bestehend im Wesentlichen aus 34,5 Massen-% (Z)-1-Chlor-2,3,3-trifluorpropen und 65,5 Massen-% trans-1,2-Dichlorethylen.

2. Azeotropartige Zusammensetzung, bestehend im Wesentlichen aus von 28 bis 41 Massen-% (Z)-1-Chlor-2,3,3-trifluorpropen und aus von 59 bis 72 Massen-% trans-1,2-Dichlorethylen.

3. Azeotropartige Zusammensetzung nach Anspruch 2, die eine relative Flüchtigkeit von 1,00±0,10 aufweist.

4. Zusammensetzung, enthaltend (Z)-1-Chlor-2,3,3-trifluorpropen und trans-1,2-Dichlorethylen, wobei der Gesamtanteil von (Z)-1-Chlor-2,3,3-trifluorpropen und trans-1,2-Dichlorethylen in der Zusammensetzung zu der Gesamtmenge der Zusammensetzung mindestens 90 Massen-% beträgt und das Massenverhältnis von (Z)-1-Chlor-2,3,3-trifluorpropen zu trans-1,2-Dichlorethylen von 28/72 bis 41/59 beträgt.

5. Zusammensetzung nach Anspruch 4, die weiter (E)-1-Chlor-2,3,3-trifluorpropen enthält.

6. Reinigungsmittel, enthaltend die azeotrope Zusammensetzung wie in Anspruch 1 definiert oder die azeotropartige Zusammensetzung wie in Anspruch 2 oder 3 definiert oder die Zusammensetzung wie in Anspruch 4 oder 5 definiert.

7. Lösungsmittel, enthaltend die azeotrope Zusammensetzung wie in Anspruch 1 definiert oder die azeotropartige Zusammensetzung wie in Anspruch 2 oder 3 definiert oder die Zusammensetzung wie in Anspruch 4 oder 5 definiert.

8. Aerosolzusammensetzung, enthaltend die azeotrope Zusammensetzung wie in Anspruch 1 definiert oder die azeotropartige Zusammensetzung wie in Anspruch 2 oder 3 definiert oder die Zusammensetzung wie in Anspruch 4 oder 5 definiert.

9. Wärmeübertragungsflüssigkeit, enthaltend die azeotrope Zusammensetzung wie in Anspruch 1 definiert oder die azeotropartige Zusammensetzung wie in Anspruch 2 oder 3 definiert oder die Zusammensetzung wie in Anspruch 4 oder 5 definiert.

## Revendications

1. Composition azéotropique essentiellement constituée de 34,5 % en masse de (Z)-1-chloro-2,3,3-trifluoropropène et 65,5 % en masse de trans-1,2-dichloroéthylène.

2. Composition de type azéotrope essentiellement constituée de 28 à 41 % en masse de (Z)-1-chloro-2,3,3-trifluoropropène et de 59 à 72 % en masse de trans-1,2-dichloroéthylène.

3. Composition de type azéotrope selon la revendication 2, qui a une volatilité relative de 1,00±0,10.

4. Composition contenant du (Z)-1-chloro-2,3,3-trifluoropropène et trans-1,2-dichloroéthylène, dans laquelle la proportion totale de (Z)-1-chloro-2,3,3-trifluoropropène et trans-1,2-dichloroéthylène dans la composition sur la quantité totale de la composition est d'au moins 90 % en masse, et le rapport massique de (Z)-1-chloro-2,3,3-trifluoropropène sur trans-1,2-dichloroéthylène va de 28/72 à 41/59.

5. Composition selon la revendication 4, qui contient en outre du (E)-1-chloro-2,3,3-trifluoropropène.

6. Détergent contenant la composition azéotropique selon la revendication 1 ou la composition de type azéotrope selon la revendication 2 ou 3 ou la composition selon la revendication 4 ou 5.

7. Solvant contenant la composition azéotropique selon la revendication 1 ou la composition de type azéotrope selon la revendication 2 ou 3 ou la composition selon la revendication 4 ou 5.

8. Composition aérosol contenant la composition azéotropique selon la revendication 1 ou la composition de type azéotrope selon la revendication 2 ou 3 ou la composition selon la revendication 4 ou 5.

9. Fluide de transfert de chaleur contenant la composition azéotropique selon la revendication 1 ou la composition de type azéotrope selon la revendication 2 ou 3 ou la composition selon la revendication 4 ou 5.
